# EUROPEAN PATENT SPECIFICATION

(11) **EP 2 049 191 B1**
(45) Date of publication and mention of the grant of the patent: **03.04.2013**
(21) Application number: 07766308.6
(22) Date of filing: 19.07.2007
(51) Int. Cl.: A61M 39/10

(54) **MALE FLUID CONNECTOR**
MÄNNLICHER FLUID VERBINDER
CONNECTEUR MÂLE POUR FLUIDE

(30) Priority: 20.07.2006 GB 0614452
(43) Date of publication of application: 22.04.2009
(73) Proprietor: International Patents Inc., Victoria, Mahé (SC)
(72) Inventor: Young Peter Jeffrey, Roydon King's Lynn, Norfolk PE32 1AR (GB)
(74) Representative: Jacob, Reuben Ellis
(86) International application number: PCT/GB2007/002743
(87) International publication number: WO 2008/009948

(56) References cited:
- WO-A-2005/055919
- GB-A- 2 383 828
- US-A- 5 286 067
- US-B1- 6 612 624

## Description

The invention provides a connector system for connecting conduits for fluid flow therebetween.

In hospitals and medical facilities, the standard method for attaching infusion equipment such as syringes and pumps to catheters and IV tubes and other similar equipment *in situ* in a patient involves the use of the Luer connector system. Luer connectors consist of male and female connector parts which mutually interengage in a fluid-tight manner, either by a simple interference fit, known as a slip or, in a variation, by a simple thread, known as a lock.

Intravenous and other medical connectors such as epidural connectors are commonly Luer-locking connections. The male Luer connector is normally on the infusion set and the female connector is normally on the catheter accessing a body cavity such as the blood stream. Connection of the two therefore allows passage of fluid from the infusion set to the patient. Tracheal tube cuff air connectors are commonly Luer slip connectors. It is important that the tubing which carries gas to a tracheal tube cuff is not accidentally connected to an intravenous or epidural connector.

A male connector according to the preamble of claim 1 is known from US 6,612,624, Fig. 8D.

It is an object of the invention to provide a male connector which will connect to a tracheal tube cuff air connector, but not to an intravenous or epidural connector.

According to the invention defined in claim 1, there is provided a male connector for use in a connector system for connecting conduits for fluid flow therebetweeen, the male connector being adapted to connect only to a standard female slip Luer connector.

The male connector comprises means for preventing connection with a standard female locking Luer connector.

The means for preventing connection comprise a barrier.

The male connector comprises a first conduit held within a sleeve, the first conduit being insertable in a second conduit provided on the female connector to form a connection between the two conduits, the barrier being positioned at the entrance of the sleeve.

The barrier is such that at least part of the sleeve has an internal cross-section small enough to prevent a standard female locking Luer connector from being fully inserted in the sleeve with the first conduit of the male connector inserted in the second conduit of the standard female locking Luer connector.

The barrier may comprise a ridge around all or part of the inner circumference of the sleeve.

The barrier may be part of the sleeve, and the shape of the sleeve may be such that a standard female locking Luer connector is prevented from being fully inserted in the sleeve with the first conduit of the male connector inserted in the second conduit of the standard female locking Luer connector.

The barrier is such that the sleeve has an internal cross-section large enough to allow a standard female slip Luer connector to be fully inserted in the sleeve with the first conduit of the male connector inserted in the second conduit of the standard female slip Luer connector.

The male connector may comprise means to guide the each female connector on insertion of the first conduit into the second conduit.

The guide means may be tapered towards the entrance of the sleeve.

The guide means may comprise the barrier.

According to claim 14, there is provided an infusion set including a male connector as defined above.

The invention will now be illustrated by way of example with reference to the following drawings, of which:
Figure 1 shows a prior art standard female locking Luer connector and a prior art standard male locking Luer connector;
Figure 2 shows a prior art standard female slip Luer connector and a prior art standard male locking Luer connector;
Figure 3 shows the connectors of Figure 1 linked;
Figure 4 shows the connectors of Figure 2 linked;
Figure 5 shows side view of a first embodiment of a male connector according to the invention;
Figure 6 shows an end view of the male connector shown in Figure 5;
Figure 7 shows a second embodiment of a male connector according to the invention;
Figure 8 shows a first modification of the male connectors shown in Figures 5 to 7;
Figure 9 shows a second modification of the male connectors shown in Figures 5 to 7; and
Figure 10 shows a third modification of the male connectors shown in Figures 5 to 7;

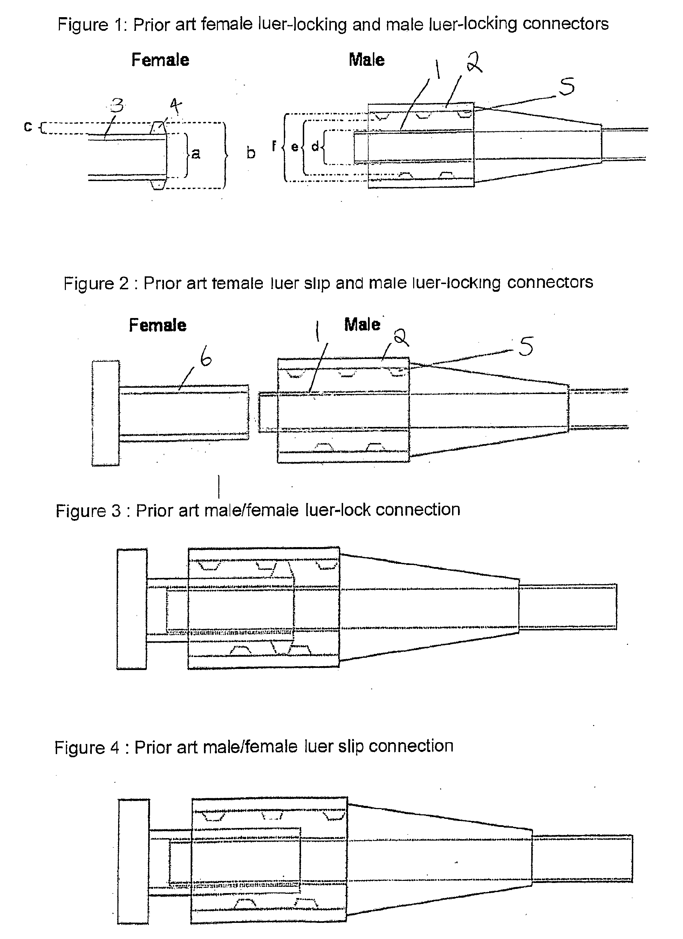

The standard male Luer locking connector shown in Figure 1 has a first conduit (1) held within a sleeve (2). The standard female Luer locking connector shown in Figure 1 has a second conduit (3). The outside of the second conduit (3) carries a number of protuberances (4). The inside of the sleeve (2) carries a screw thread (5). In use, the first conduit (1) is inserted in the second conduit (3) to form a fluid-tight connection between the two connectors, and the protuberances (4) of the standard female Luer connector engage the screw thread (5) of the standard male Luer connector (2) as shown in Figure 3.

The standard male Luer locking connector shown in Figure 2 also has a first conduit (1) held within a sleeve (2). The standard female Luer slip connector shown in Figure 2 has a second conduit (6), but no protuberances. In use, the first conduit (1) is inserted in the second conduit (6) to form a fluid-tight connection between the two connectors as shown in Figure 4.

The dimensions a-f of current standard Luer connectors are indicated in European standard EN1707 of January 1997.

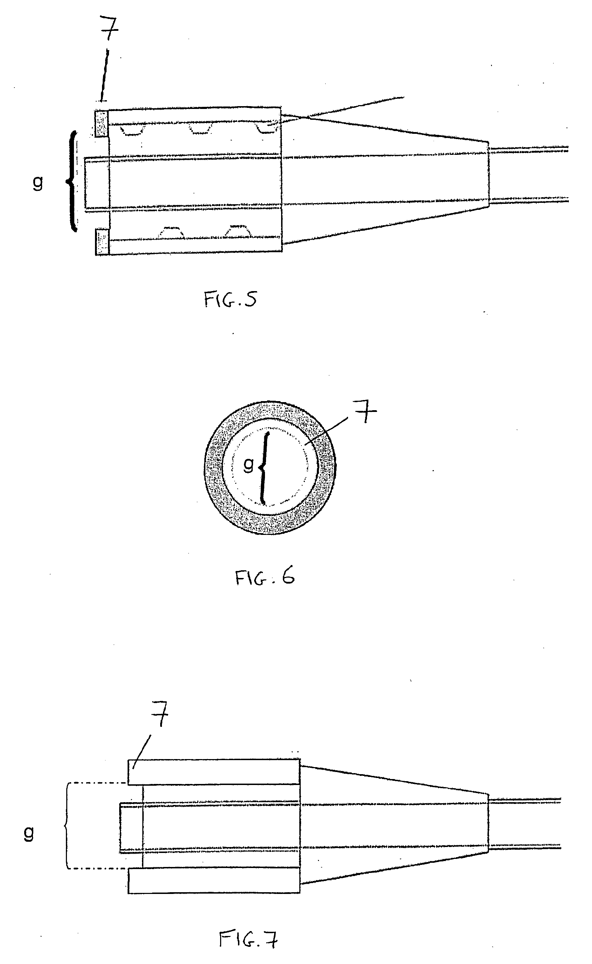

Figures 5 to 7 show two embodiments of the present invention.

Each embodiment comprises a male Luer connector with an additional disc (7) at the entrance to the sleeve (2). The diameter of the disc (7) is such that dimension g is smaller than dimension b of the standard female Luer lock connector. This means that that the protruberances (4) of the standard female Luer lock connector are unable to enter into the sleeve (2) of the connector of the invention, with the result that thestandard female Luer lock connector is unable to connect with the connector of the invention. However, dimension g is not so small that it prevents connection of the prior art Luer slip connector.

To prevent attachment, dimension g must only be smaller than dimension b for enough of the circumference of the connector to prevent the protruberances (4) of the standard female Luer lock connector from entering into the sleeve (2) of the connector of the invention. Accordingly, there are many other possible arrangements. For example, the disc may extend around only part of the circumference of the sleeve, or the cross-section of the sleeve may be non-cylindrical.

The connector may be coloured to assist the user in identifying that it is not intended for intravenous connection.

The connector may have a clip attached designed to engage in a groove, ridge, notch or protuberance on the outer surface of the female slip connector to secure the connector in place.

The connector may be attached to a syringe. In prior art, a syringe can have a male slip tip end connector or a male luer-locking end connector. The current embodiment has a sleeve which prevents connection to a female luer-locking connector.

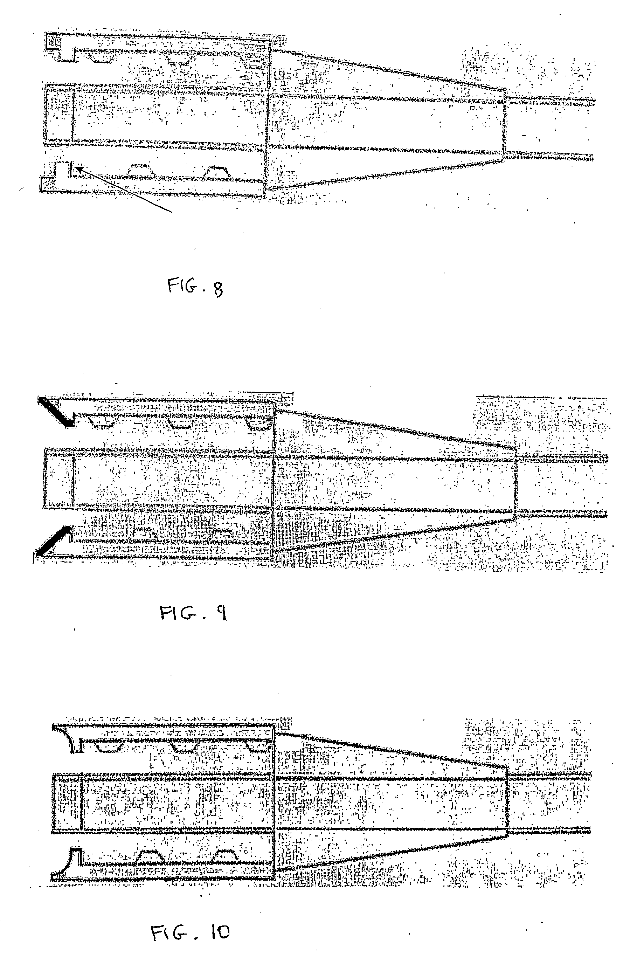

The male connector may have a ridge modification or indentation modification at the entrance to the connector. The ridge or indentation helps to guide the female connector into place. This can have perpendicular straight (Figures 8 and 9) or curved edges (Figure 10) or edges with other surface configurations.

Another embodiment of the rim modification comprises radial (Figure 11) or circumferential (Figure 12) slopes as shown by the arrows. This sloping helps to improve alignment in embodiments where the barrier does not extend around the entire circumference of the sleeve (2). The circumferential slopes cause a wave profile with nadirs at the gaps (11) and peaks at the midpoints (10).

Another embodiment of the rim modification comprises a combination of both radial and circumferential slopes that can improve alignment.

Another embodiment of the rim modification comprises a radial and/or circumferential slope on the inner surface (Figure 12). This helps disengagement of the attached connector.

Any individual, combination or all of these embodiments can be used together to improve the alignment of the connector.

## Claims

1. A male connector for use in a connector system for connecting conduits for fluid flow therebetween, the male connector comprising a first conduit held within a sleeve having a first internal diameter and a barrier (7) positioned at an entrance of the sleeve to at least partially reduce the first internal diameter of the sleeve, the barrier having a second internal diameter (g) that is less than the first internal diameter of the sleeve for at least enough of the circumference of the connector to prevent the protuberances (4) of a standard female locking luer connector from entering into the sleeve (2) such that that the male connector is **characterised in that** the male connector can only connect to a standard female slip Luer connector and prevents connection of a standard female locking Luer connector.

2. A male connector according to claim 1, wherein a screw thread is positioned on an inside surface of the sleeve.

3. A male connector according to claim 1 or 2, wherein the barrier is connected to the sleeve.

4. A male connector according to claims 1 or 2, wherein the barrier is integral to the sleeve.

5. A male connector according to claim 4, wherein the barrier comprises a ridge around all or part of the inner circumference of the sleeve.

6. A male connector according to claim 5, wherein the ridge comprises radial slopes.

7. A male connector according to claim 5, wherein the ridge comprises circumferential slopes.

8. A male connector according to claim 5, wherein the ridge comprises both radial slopes and circumferential slopes

9. A male connector according to claims 2 or 2, wherein the barrier is part of the sleeve, and the shape of the sleeve is such that a standard female locking Luer connector is prevented from being fully inserted in the sleeve with the first conduit of the male connector inserted in the second conduit of the standard female locking Luer connector.

10. A male connector according to any one of the preceding claims further comprising means for guiding the female connector on insertion of the female connector into the sleeve.

11. A male connector according to claim 10, wherein the guide means is tapered towards the entrance of the sleeve.

12. A male connector according to claim 11, wherein the guide means comprises the barrier.

13. A male connector according to any of the preceding claims, wherein the barrier comprises a disc and the at least one aperture is a notch in the circumference of the disc.

14. An infusion set including a male connector according to any of claims 1 to 13.

## Patentansprüche

1. Schraubverbindung mit Außengewinde zur Verwendung in einem Verbindersystem zum Verbinden von Leitungen für Fluidstrom dazwischen, wobei die Schraubverbindung mit Außengewinde eine erste Leitung umfasst, die in einer Manschette gehalten wird, welche einen ersten Innendurchmesser und eine Sperre (7) hat, die sich am Eingang der Manschette befindet, um zumindest teilweise den ersten Innendurchmesser der Manschette zu reduzieren, wobei die Sperre einen zweiten Innendurchmesser (g) hat, der kleiner als der erste Innendurchmesser der Manschette ist, der zumindest groß genug für den Umfang der Schraubverbindung ist, um die Ausstülpungen (4) eines Standard-Luer-Lock-Verbinders mit Innengewinde daran zu hindern, in die Manschette (2) cinzudringen, derart dass, die Schraubverbindung mit Außengewinde **dadurch gekennzeichnet ist, dass** die Schraubverbindung mit Außengewinde sich nur mit einer Standard-Slip-Luer-Schraubverbindung mit Innengewinde verbinden kann und den Anschluss eines Standard-Luer-Lock-Verbinders mit Innengewinde verhindert.

2. Schraubverbindung mit Außengewinde nach Anspruch 1, wobei ein Schraubgewinde sich auf einer Innenfläche der Manschette befindet.

3. Schraubverbindung mit Außengewinde nach Anspruch 1 oder 2, wobei die Sperre mit der Manschette verbunden ist.

4. Schraubverbindung mit Außengewinde nach Anspruch 1 oder 2, wobei die Sperre in die Manschette integriert ist.

5. Schraubverbindung mit Außengewinde nach Anspruch 4, wobei die Sperre eine Kante rings um den ganzen inneren Umfang der Manschette oder einen Teil derselben umfasst.

6. Schraubverbindung mit Außengewinde nach Anspruch 5, wobei die Kante radiale Schrägen umfasst.

7. Schraubverbindung mit Außengewinde nach Anspruch 5, wobei die Kante umlaufende Schrägen umfasst.

8. Schraubverbindung mit Außengewinde nach Anspruch 5, wobei die Kante sowohl Knotenflanken wie auch umlaufende Schrägen umfasst.

9. Schraubverbindung mit Außengewinde nach Anspruch 1 oder 2, wobei die Sperre Teil der Manschette ist und die Form der Manschette derart ist, dass ein Standard-Luer-Lock-Verbinder mit Innengewinde daran gehindert wird, vollständig in die Manschette eingeführt zu werden, wobei die erste Leitung der Schraubverbindung mit Außengewinde in die zweite Leitung des Standard-Luer-Lock-Verbinders mit Innengewinde eingeführt ist.

10. Schraubverbindung mit Außengewinde nach einem der vorherigen Ansprüche, die ferner Mittel zum Führen der Schraubverbindung mit Innengewinde beim Einführen der Schraubverbindung mit Innengewinde in die Manschette umfasst.

11. Schraubverbindung mit Außengewinde nach Anspruch 10, wobei das Fülhrungsmittel zum Eingang der Manschette hin verjüngt ist.

12. Schraubverbindung mit Außengewinde nach Anspruch 11, wobei das Führungsmittel die Sperre umfasst.

13. Schraubverbindung mit Außengewinde nach einem der vorherigen Ansprüche, wobei die Sperre eine Scheibe umfasst und die mindestens eine Öffnung eine Kerbe im Umfang der Scheibe ist.

14. Infusionsset, das eine Schraubverbindung mit Außengewinde nach einem der Ansprüche 1 bis 13 umfasst.

## Revendications

1. Connecteur mâle destiné à être utilisé dans un système de connecteur pour raccorder des conduits pour l'écoulement de fluide entre eux, le connecteur mâle comprenant un premier conduit maintenu à l'intérieur d'un manchon ayant un premier diamètre interne et une barrière (7) positionnée à l'entrée du manchon afin de réduire au moins partiellement le premier diamètre interne du manchon, la barrière ayant un second diamètre interne (g) qui est inférieur au premier diamètre interne du manchon sur au moins une partie suffisante de la circonférence du connecteur pour empêcher les protubérances (4) d'un raccord Luer femelle standard d'entrer dans le manchon (2) de sorte que le connecteur mâle est **caractérisé en ce que** le connecteur mâle peut uniquement se raccorder à un connecteur Luer coulissant femelle standard et empêcher le raccordement d'un connecteur Luer femelle standard à verouillage.

2. Connecteur mâle selon la revendication 1, dans lequel un filetage de vis est positionné sur une surface intérieure du manchon.

3. Connecteur mâle selon la revendication 1 ou 2, dans lequel la barrière est raccordée au manchon.

4. Connecteur mâle selon les revendications 1 ou 2, dans lequel la barrière est solidaire du manchon.

5. Connecteur mâle selon la revendication 4, dans lequel la barrière comprend une crête autour de la totalité ou d'une partie de la circonférence interne du manchon.

6. Connecteur mâle selon la revendication 5, dans lequel la crête comprend des inclinaisons radiales.

7. Connecteur mâle selon la revendication 5, dans lequel la crête comprend des inclinaisons circonférentielles.

8. Connecteur mâle selon la revendication 5, dans lequel la crête comprend à la fois des inclinaisons radiales et des inclinaisons circonférentielles.

9. Connecteur mâle selon les revendications 1 ou 2, dans lequel la barrière fait partie du manchon, et la forme du manchon est telle que l'insertion complète du connecteur Luer femelle standard est empêchée dans le manchon avec le premier conduit du connecteur mâle inséré dans le second conduit du connecteur Luer femelle standard.

10. Connecteur mâle selon l'une quelconque des revendications précédentes, comprenant en outre des moyens pour guider le connecteur femelle suite à l'insertion du connecteur femelle dans le manchon.

11. Connecteur mâle selon la revendication 10, dans lequel les moyens de guidage sont progressivement rétrécis vers l'entrée du manchon.

12. Connecteur mâle selon la revendication 11, dans lequel les moyens de guidage comprennent une barrière.

13. Connecteur mâle selon l'une quelconque des revendications précédentes, dans lequel la barrière comprend un disque et la au moins une ouverture est une encoche dans la circonférence du disque.

14. Ensemble de perfusion comprenant un connecteur mâle selon l'une quelconque des revendications 1 à 13.
